# EUROPÄISCHE PATENTSCHRIFT

(11) **EP 0 917 879 B1**
(45) Veröffentlichungstag und Bekanntmachung des Hinweises auf die Patenterteilung: **17.07.2002**
(21) Anmeldenummer: 98121684.9
(22) Anmeldetag: 13.11.1998
(51) Int. Cl.: A61K 47/00, A61K 47/02, A61K 47/42, C12N 9/96

(54) **Verbessertes Verfahren zur Stabilisierung von Proteinen**
Improved process for protein stabilization
Procédé amélioré pour la stabilisation des protéines

(30) Priorität: 22.11.1997 EP 97120528; 19.02.1998 EP 98102846
(43) Veröffentlichungstag der Anmeldung: 26.05.1999
(73) Patentinhaber: Roche Diagnostics GmbH, 68305 Mannheim (DE)
(72) Erfinder: Hellerbrand, Klaus, 82269 Geltendorf (DE); Papadimitriou, Apollon, 83673 Bichl (DE); Winter, Gerhard, 69221 Dossenheim (DE)
(74) Vertreter: Notegen, Eric-André

(56) Entgegenhaltungen:
- EP-A- 0 852 951
- DATABASE WPI Week 9847 Derwent Publications Ltd., London, GB; AN 98-555428 XP002096028 "Freeze-dried immunobiological prepn. stabiliser compsn. contg. peptone, bacteriostatic, sucrose and phosphate buffer, with additional beef serum albumin, EDTA and potassium chloride" & RU 2 109 290 C (VEKTOR RES. CENTER) , 20. April 1998
- CHEMICAL ABSTRACTS, vol. 127, no. 14, 6. Oktober 1997 Columbus, Ohio, US; abstract no. 195334, M C HELLER ET AL.: "Manipulation of lyophilization-induced phase separation: implication for pharmaceutical proteins" XP002096027 & BIOTECHNOL. PROGR., Bd. 13, Nr. 5, Mai 1997, Seiten 590-596,

## Beschreibung

Gegenstand der Erfindung ist ein verbessertes Verfahren zur Stabilisierung von Proteinen beim Einfrier- oder Lyophilisationsprozeß oder während der Lagerung bei niedrigen Temperaturen.

Proteine, wie beispielsweise Enzyme oder Antikörper sowie Fragmente davon, sind in wäßrigen Lösungen und bei Lagerung bei niedrigen Temperaturen (unter 0°C) und insbesondere bei wiederholten Einfrier- und Auflauprozessen instabil und neigen zu Aktivitätsverlust und/oder zur Bildung löslicher und unlöslicher Aggregate, wobei sich letzteres durch die Bildung von Partikeln und damit in Form von Trübungen bemerkbar macht. Für pharmazeutische Zusammensetzungen von Proteinen sind solche Aggregat- und/oder Partikelbildungen jedoch nicht oder zumindest nur in Spuren tolerierbar. Eine pharmazeutische Zusammensetzung sollte eine klare Lösung darstellen und auch dann, wenn sie als Lyophilisat vorliegt, bei der Rekonstitution zu einer klaren partikelfreien Lösung, die auch frei von löslichen Proteinaggregaten ist, führen.

Zur Stabilisierung von Proteinen in Lösungen sind eine Vielzahl von Verfahren und Zusätzen bekannt. Beispielsweise ist in der EP-A 0 599 344 die Stabilisierung von Proteinen durch Zusatz von Heat shock Proteinen, wie HSP25, beschrieben. In der EP-A 0 318 081 ist die Stabilisierung von Antikörpern durch Zusatz von Blockpolymeren aus Polyoxypropylen und Polyoxyethylen und durch Phospholipide beschrieben. In der EP-A 0 025 275 wird die Stabilisierung von Immunoglobulinen durch Zusatz eines Salzes einer basischen stickstoffhaltigen Substanz, wie beispielsweise Arginin, Guanidin oder Imidazol, beschrieben. Ebenfalls geeignete Zusätze zur Stabilisierung sind Polyether (EP-A 0 018 609), Glycerin, Albumin und Dextransulfat (US-Patent 4,808,705), Detergentien, wie Tween®20 (DE 26 52 636, GB 8514349), Chaperone, wie GroEL (Mendoza, J.A. Biotechnol. Tech. 10 (1991) 535-540), Citratpuffer (WO 93/22335) oder Chelatbildner (WO 91/15509). Mit diesen Zusätzen gelingt es zwar, Proteine in gewissem Umfang in wäßrigen Lösungen zu stabilisieren. Es hat sich jedoch gezeigt, daß keines der aus dem Stand der Technik bekannten Verfahren geeignet ist, Proteine bei wiederholten Einfrier- und Auflauprozessen so zu stabilisieren, daß keine oder für therapeutische Zwecke vernachlässigbare lösliche oder unlösliche Proteinaggregate beim Wiederauftauen, bei der Lagerung bei Temperaturen unter 0°C bzw. bei einer nach Lyophilisation erfolgten Rekonstitution der Lösung entstehen.

In der EP-A 0 314 095 wird ein Lyophilisat eines Plasmaproteins wie beispielsweise Faktor VIII beschrieben, welches als Puffersubstanz Histidinpuffer und als Zusatzstoff Calciumchlorid enthält und in hoher Ionenstärke (0,35 bis 1,2 mol/l NaCl) vorliegt.

In der EP-A 0 315 968 wird ein Lyophilisat eines Plasmaproteins wie beispielsweise Faktor VIII beschrieben, welches 0,5 bis 15 mmol/l Natrium- oder Kaliumchlorid, 0,01 bis 10 mmol/l Lysinhydrochlorid und 0,2 bis 5 mmol/l Histidin als Pufferion enthält. Histidinpuffer ist jedoch nicht geeignet, Proteine zu stabilisieren und eine Aggregat- und Partikelbildung bei der Rekonstitution von Lyophilisaten von Proteinen zu verhindern.

M. Heller et al. beschreibt in Biotechnol. Prog. 13 (1997) 590 - 596 die Optimierung der Lyophilisation für polyethylenglykolhaltige Lösungen von Hämoglobin zur Vermeidung von Phasentrennungen durch Ersatz von NaCl durch KCl.

EP-A 0 852 951 beschreibt lyophilisierte pharmazeutische Zubereitungen von Antikörpern, die Kaliumphosphatpuffer und NaCl enthalten. Einen Hinweis darauf, wie Aggregatbildungen vermieden werden könnten, enthält diese Publikation jedoch nicht. Aufgabe der Erfindung ist demzufolge die Bereitstellung eines Verfahrens, mit dem eine Aggregat- und Partikelbildung bei der Rekonstitution von Lyophilisaten pharmazeutischer Zusammensetzungen von Proteinen weitgehend verhindert werden kann.

Gegenstand der Erfindung ist demnach ein verbessertes Verfahren zur Verhinderung der Bildung von Proteinaggregaten in einer aus einem Lyophilisat rekonstitutierten Lösung einer pharmazeutischen Zusammensetzung eines Proteins, vorzugsweise eines Antikörpers, wobei eine wäßrige gepufferte Lösung des Proteins eingefroren, aufgetaut, in Kompartimente von injizierbaren Mengen zerteilt wird und diese Kompartimente lyophilisiert werden, welches dadurch gekennzeichnet ist, daß die wäßrige gepufferte Lösung des Proteins Kaliumphosphatpuffer als Puffersubstanz enthält und das Verhältnis Kalium- zu Natriumionen in der Lösung 10: 1 oder größer ist. Vorzugsweise enthält die wäßrige gepufferte Lösung im wesentlichen keine Natriumionen.

Die Erfindung eröffnet die Möglichkeit, pharmazeutische Zusammensetzungen von Proteinen, insbesondere von Proteinen, die zur Dimerisierung und Multimerisierung neigen, wie Antikörper, im neutralen pH-Bereich (pH 6 - 8, vorzugsweise pH 6,5 - 7,5), in einer stabilen pharmazeutischen Zusammensetzung zu formulieren. Im neutralen pH-Bereich neigen Proteine wie Antikörper zur Aggregation, insbesondere, wenn die Lösungen ein- oder mehrfach eingefroren (ggf. lyophilisiert) und wieder aufgetaut werden.

Insbesondere vorteilhaft ist eine pharmazeutische Zusammensetzung in Kaliumphosphatpuffer im pH-Bereich zwischen 6 und 8, bei einer Pufferkonzentration zwischen 10 und 300 mmol/l, vorzugsweise zwischen 50 und 250 mmol/l, wobei möglichst wenig Natriumionen in der pharmazeutischen Zusammensetzung enthalten sind und die Zusammensetzung kein Polyethylenglycol enthält. Zweckmäßig beträgt das Verhältnis von Kalium- zu Natriumionen in der Lösung 10 : 1 oder mehr. Besonders bevorzugt wird in der pharmazeutischen Zusammensetzung als Puffersubstanz Kaliumphosphatpuffer allein verwendet und kein Natriumsalz (wie z.B. Natriumchlorid) zugesetzt. In einem solchen Fall sind praktisch keine Natriumionen in der pharmazeutischen Zusammensetzung enthalten, oder sie sind nur in so geringen Mengen enthalten, daß sie keine Bildung von Aggregaten der Proteine beim wiederholten Einfrieren oder Auftauen verursachen.

Es hat sich gezeigt, daß Lyophilisate aus Proteinlösungen, welche während des Herstellprozesses mindestens einmal eingefroren werden, sich dann weitgehend ohne Bildung von Trübungen rekonstituieren lassen, wenn als Puffersubstanz Kaliumphosphatpuffer verwendet wird. Die üblicherweise verwendeten Puffer, wie Natriumphosphatpuffer, Histidinpuffer oder Citratpuffer, führen bei einem solchen Verfahren zur Bildung von Aggregaten, hauptsächlich zusammengesetzt aus dem Protein, und damit auch zu Trübungen in erheblichem Umfang. Die eingefrorenen Proteinlösungen sind bereits ab ca. -15°C völlig durchgefroren, haben Eutektika oberhalb ca. -15°C, und können somit bereits bei dieser Temperatur bzw. bei tieferen Temperaturen, zweckmäßig z.B. bei -20°C, gelagert werden. Da eine Lösung erst unterhalb der eutektischen Temperatur vollständig durchgefroren ist, bedeutet dies, daß ein Protein in einem Na-Ionen-haltigen Phosphatpuffer während der Gefrierlagerung (üblicherweise bei -20°C) und während des Einfrier-/Auftauvorgangs einem höheren Streß ausgesetzt ist als im Na-Ionen-freien Puffer oder in einem Puffer, in dem die Na-Ionen-Konzentration sehr gering ist. Erfindungsgemäß wird dieser Streß in den oben genannten Formulierungen vermieden, wodurch die Aggregat- bzw. Partikelbildung unterdrückt wird. Diese Formulierung ermöglicht eine stabile Lagerung der Proteinlösung bei -20°C, wodurch Kosten gespart werden können. Kaliumphosphatpuffer zeigen im Gegensatz zu Natriumphosphatpuffer nur einen geringfügigen pH-Shift (vorzugsweise höchstens ± 1 pH-Einheiten, besonders bevorzugt höchstens ± 0,5 pH-Einheiten) während des Einfriervorgangs.

Es hat sich gezeigt, daß die Konzentration des Phosphatpuffers mindestens 10 mmol/l betragen sollte, vorzugsweise etwa 50 mmol/l oder höher sein sollte, um die Partikelbildung wirksam zu verhindern. Da in pharmazeutischen Zusammensetzungen (vorzugsweise also in der rekonstituierten Lösung) die Osmolarität nicht zu hoch sein darf (sie sollte zweckmäßig im physiologischen Bereich liegen, vorzugsweise nach Rekonstitution ca. 300 mOsm (± 20 mOsm, geeignet ist auch ein Bereich von 100 bis 500 mOsm) betragen), sollte zweckmäßig die Konzentration der Puffersubstanz oder gegebenenfalls die Summe an Puffersubstanz und Salz nicht mehr als 250 - 300 mmol/l betragen. Vorzugsweise beträgt die Pufferkonzentration zwischen 50 und 250 mmol/l im Kompartiment. In den zur Herstellung der Kompartimente verwendeten Lösungen (bulkware) sind jedoch höhere Konzentrationen an Puffersubstanz und/oder Salz tolerierbar.

Falls ein Salzzusatz in der pharmazeutischen Zusammensetzung, insbesondere zur Einstellung der Ionenstärke, gewünscht ist, ist es erfindungsgemäß vorteilhaft, ebenfalls auf Natriumsalze zu verzichten oder die Konzentration der Natriumionen wesentlich geringer als die Konzentration der Kaliumionen zu wählen. Anstelle des sonst üblichen Natriumchlorids, wird deshalb zweckmäßig ein Kaliumsalz, wie beispielsweise Kaliumchlorid, zugesetzt. Es hat sich allerdings gezeigt, daß geringe Mengen an Natriumsalzen (z.B. ca. 10 mmol/l oder darunter) nicht störend wirken, solange das Verhältnis Kaliumionen zu Natriumionen 10 : 1 oder größer ist. Der Zusatz von Calciumsalzen wie z.B. Calciumchlorid ist nicht möglich, da durch einen solchen Zusatz Calciumphosphat ausgefällt wird und damit neben der Bildung von unerwünschten Trübungen die Pufferwirkung des erfindungsgemäß verwendeten Kaliumphosphatpuffers aufgehoben wird.

Unter nichtlöslichen Aggregaten, deren Bildung im erfindungsgemäßen Verfahren verhindert werden soll, sind im wesentlichen Proteinaggregate zu verstehen, deren Größe üblicherweise bei mindestens 1 µm, aber durchaus auch im Bereich über 10 µm liegt. Die Bestimmung der Partikel kann durch geeignete Partikelzählverfahren mit handelsüblichen Partikelzählgeräten, wie z.B. Partikelzählgerät AccuSizer 700 von PSS (Particle Sizing Systems, USA), durchgeführt werden. Erfindungsgemäß ist eine Verbesserung des Verfahrens erreicht, wenn die Anzahl der Partikel zwischen 2 und 400 µm/ml bei <3.000 oder die Anzahl der Partikel zwischen 10 und 400 µm/ml bei 2.000 oder darunter liegt. Gemäß USP (US-Pharmacopoe) dürfen pro injizierter Dosis eines Arzneimittels maximal 6000 Partikel im Bereich größer 10 µm und maximal 600 Partikel im Bereich größer 25 um vorliegen. Dies kann erfindungsgemäß auf einfache Weise für therapeutische Zusammensetzungen von Proteinen erreicht werden.

Unter Proteinen (Polypeptiden) im Sinne der Erfindung sind Proteine oder Proteinfragmente sowie chemisch modifizierte Proteine zu verstehen. Proteine, deren Stabilisierung fiir pharmazeutische Formulierungen wünschenswert sind, sind vorzugsweise Antikörper, Antikörperfusionsproteine wie Immuntoxine, Enzyme und Proteinhormone wie Erythropoietin, Somatostatin, Insulin, Cytokine, Interferone oder Plasminogenaktivatoren.

Unter Kompartimenten im Sinne der Erfindung sind Teilmengen der Proteinlösung zu verstehen, die gegebenenfalls nach Weiterverarbeitung (Zusatz weiterer pharmazeutisch verträglicher Stoffe) als pharmazeutische Zusammensetzungen vorzugsweise zur Injektion am Patienten geeignet sind.

Der pH-Bereich, in dem die pharmazeutische Zusammensetzung durch den Kaliumphosphatpuffer stabilisiert wird, ist vorzugsweise ein leicht saurer, neutraler oder leicht alkalischer Bereich (ca. pH 6 - 8, vorzugsweise um pH 7).

Vorzugsweise wird erfindungsgemäß ein nichtionisches Detergens wie Polysorbat (z.B. Tween® 80), vorzugsweise in einer Konzentration von höchstens 0,1 Gew.% und mindestens 0,01 Gew.%, zugesetzt.

Weiter bevorzugt ist es, Kryoprotektoren oder Glasbildner, wie einen nicht-reduzierenden Zucker (vorzugsweise Saccharose oder Trehalose), zuzusetzen, zweckmäßig in einer Konzentration von mindestens 10 mg/ml, vorzugsweise von ca. 30 - 100 mg/ml.

Ein weiterer Gegenstand der Erfindung ist demnach eine aggregatarme, schmelzbare feste Lagerform eines Proteins, welche amorph ist, kein Polyethylenglycol enthält und aus einer eingefrorenen Lösung des Proteins und Kaliumphosphatpuffer als wesentliche Puffersubstanz besteht, wobei in der Lösung das Verhältnis Kaliumionen zu Natriumionen mindestens 10 : 1 ist.

Unabhängig von der Konzentration der Kaliumionen und vom Restgehalt an Natriumionen, sollte das Verhältnis Kalium- zu Natriumionen mindestens 10 : 1, vorzugsweise mindestens 50 : 1 betragen. Besonders bevorzugt wird im wesentlichen Natriumionen-freier Kaliumpuffer verwendet.

In einer weiteren bevorzugten Ausführungsform der Erfindung enthält die pharmazeutische Zusammensetzung ein Protein, welches durch eine in vitro-Zellkultur (beispielsweise rekombinante Herstellung oder Kultur einer Hybridomzellinie zur Produktion von monoklonalen Antikörpern) hergestellt wurde. In diesem Fall ist es zweckmäßig, entweder beim ersten Zusatz von Salz und/oder Puffer Kaliumsalz und/oder Kaliumphosphatpuffer zuzusetzen oder zu einem späteren Zeitpunkt im Isolierungs- und Aufreinigungsprozeß eine Umpufferung durchzuführen. Dadurch wird die Polypeptidpräparation unter 0°C stabil zwischenlagerbar. Unter einer Umpufferung ist ein Austausch der Ionen, beispielsweise durch Dialyse, zu verstehen. Im Reinigungs- und Isolierungsprozeß des Proteins kann die Puffer- bzw. Salzkonzentration vor der Kompartimentierung durchaus höher als 50 - 100 mmol/l sein, da diese Zusammensetzungen nicht therapeutisch angewendet werden. Es ist jedoch wesentlich, daß vor der Kompartimentierung die für eine injizierbare Zusammensetzung geeignete Osmolarität eingestellt wird.

Die folgenden Beispiele, Publikationen und Abbildungen erläutern die Erfindung, deren Schutzumfang sich aus den Patentansprüchen ergibt, weiter. Die beschriebenen Verfahren sind als Beispiele zu verstehen, die auch noch nach Modifikationen den Gegenstand der Erfindung beschreiben.
- **Fig. 1**: zeigt die Bestimmung der Eutektika verschiedener Puffer und Salzlösungen.
- **Fig. 2**: zeigt die Verschiebung des pH-Werts während des Einfrierens von Phosphatpuffern.
- **Fig. 3**: zeigt die Partikelbildung von Lösungen eines Antikörpers (gegen L-Selectin) in verschiedenen Pufferlösungen (A, B, C) nach Scher- bzw. Einfrier-/Auftaustreß.
A: AK in 10 mmol/l KP, 150 mmol/l NaCl, pH 7; B: AK in 100 mmol/l KP, pH 7,2; C: AK in 100 mmol/l KP, 0,01 Gew.% Tween® 80, pH 7,2; a: zentrifugiert (Ausgangsmaterial); b: nach Scherstreß (30 s vortexen); c: nach sechs Einfrier-/Auftauzyklen (-20°C).
- **Fig. 4**: zeigt die Partikelbildung von Lösungen eines Antikörpers gegen HBV in verschiedenen Pufferlösungen (A, B, C) nach Scher- bzw. Einfrier-/Auftaustreß.
A: AK in 10 mmol/l KP, 30 mmol/l NaCl, pH 6,5; B: AK in 100 mmol/l KP, pH 7,2; C: AK in 100 mmol/l KP, 0,01 Gew.% Tween® 80, pH 7,2.
- **Fig. 5**: zeigt die Size Exclusion HPLC-Analyse löslicher Aggregate in Proteinlösungen (humanisierter IgG gemäß Beispiel 3) nach Lagerung bei Temperaturen unterhalb 0°C.
A: AK in 10 mmol/l KP, 150 mmol/l NaCl, pH 7,0; B: AK in 100 mmol/l KP, pH 7,2.

### Beispiel 1

### Eutektische Temperaturen verschiedener Puffer und Salzlösungen

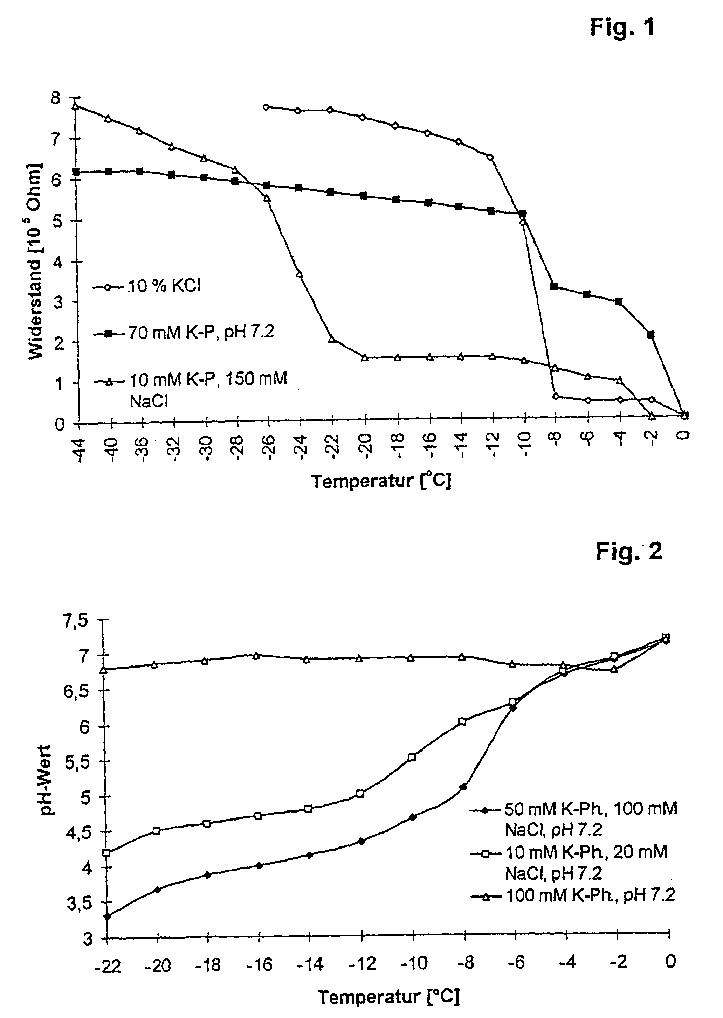

Aus Fig. 1 wird deutlich, daß die eutektische Temperatur NaCl-haltiger Puffer im Gegensatz zu den NaCl-freien Puffern oder zu den Lösungen, die KCl anstelle von NaCl enthalten, um ca. 10°C tiefer liegt. Da eine Lösung erst unterhalb der eutektischen Temperatur vollständig durchgefroren ist, bedeutet dies, daß ein Protein in einem NaCl-haltigen Phosphatpuffer während der Gefrierlagerung (üblicherweise bei -20°C) und während des Einfrier-/Auftauvorgangs einem höheren Streß ausgesetzt ist als im NaCl-freien Puffer. Erfindungsgemäß wird dieser Streß in den oben genannten Formulierungen vermieden, wodurch die Aggregat- bzw. Partikelbildung unterdrückt wird. Diese Formulierung ermöglicht eine stabile Lagerung der Proteinlösung bei -20°C, wodurch Kosten gespart werden können.

### Beispiel 2

### Verschiebung des pH-Wertes während des Einfrierens von Phosphatpuffern

Aus Fig. 2 wird deutlich, daß in NaCl-haltigen Phosphatpuffem der pH-Wert während des Einfriervorgangs aufgrund von präzipitierendem Dinatriumhydrogenphosphat stark abnimmt. Im NaCl-freien Kaliumphosphatpuffer bleibt der pH-Wert weitgehend konstant.

### Beispiel 3

### Partikelbildung in Proteinlösungen nach Scher- bzw. Einfrier-/Auftaustreß

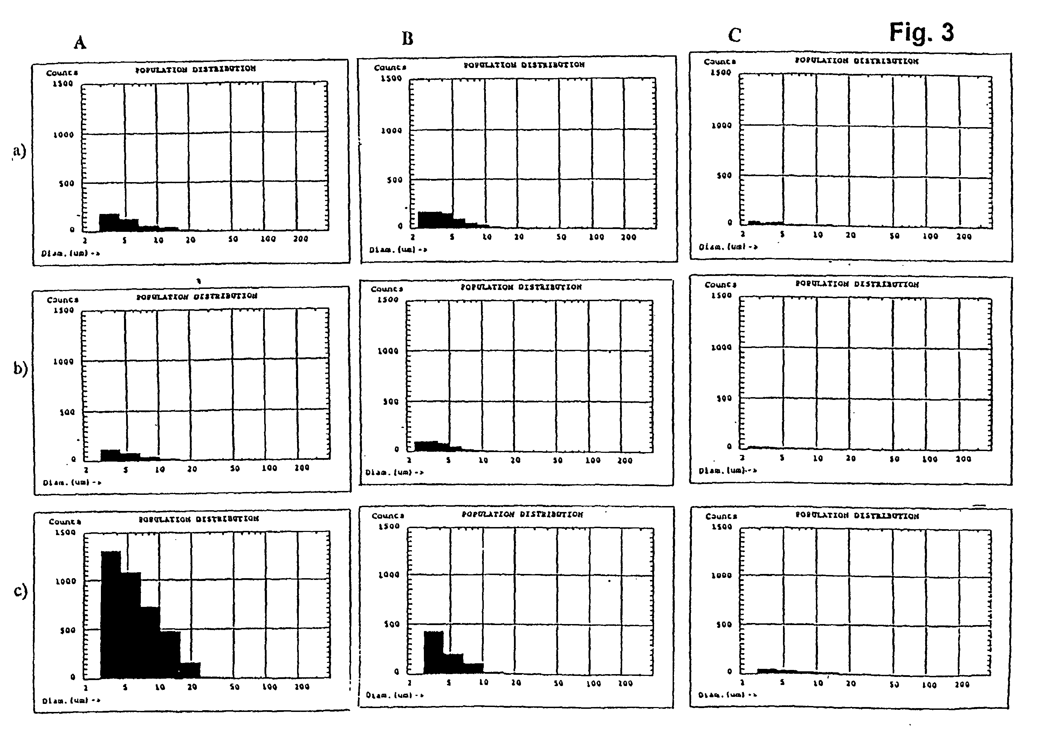

Lösungen eines humanisierten IgG (Antikörper gegen L-Selectin) in verschiedenen Puffern (A, B, C) wurden auf Partikelgehalt analysiert (Accu Sizer, Particle Sizing Systems, USA):
A) AK in 10 mmol/l KP, 150 mmol/l NaCl, pH 7
B) AK in 100 mmol/l KP, pH 7,2
C) AK in 100 mmol/l KP, 0,01 Gew.% Tween® 80, pH 7,2
   a) zentrifugiert (Ausgangsmaterial)
   b) nach Scherstreß (30 s vortexen)
   c) nach sechs Einfrier-/Auftauzyklen (-20°C).
Die Angaben in Fig. 3 beziehen sich jeweils auf 0,7 ml Probe.

Aus Fig. 3 wird deutlich, daß die Partikelbildung durch Verwendung von natriumfreien Kaliumphosphatpuffem erfindungsgemäß unterdrückt wird. Dieser Effekt kann durch die Zugabe eines nichtionischen Detergens (Tween® 80, 0,01 Gew.%) gesteigert werden.

### Beispiel 4

### Partikelbildung in Proteinlösungen nach Scher- bzw. Einfrier-/Auftaustreß

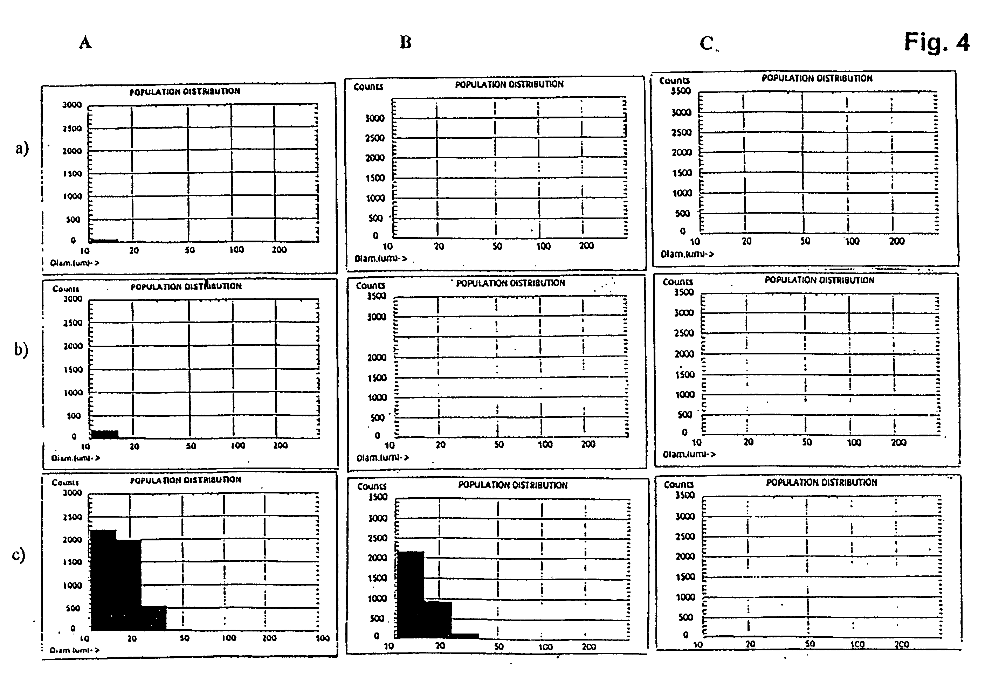

Lösungen eines Antikörpers gegen HBV in verschiedenen Puffern (A, B, C) wurden auf Partikelgehalt analysiert (Accu Sizer, Particle Sizing Systems):
A) AK in 10 mmol/l KP, 30 mmol/l NaCl, pH 6,5
B) AK in 100 mmol/l KP, pH 2
C) AK in 100 mmol/l KP, 0,01 Gew.% Tween® 80, pH 7,2
   a) zentrifugiert (Ausgangsmaterial)
   b) nach Scherstreß (30 s vortexen)
   c) nach sechs Einfrier-/Auftauzyklen (-20°C).
Die Angaben in Fig. 4 beziehen sich jeweils auf 0,7 ml Probe.

Aus Fig. 4 wird deutlich, daß die Partikelbildung durch Verwendung von natriumfreien Kaliumphosphatpuffern erfindungsgemäß unterdrückt wird. Dieser Effekt kann durch die Zugabe eines nichtionischen Detergens gesteigert werden.

### Beispiel 5

### Verhinderung der Ausbildung löslicher Aggregate während der Lagerung von Protein lösungen (humanisiertes IgG gemäß Beispiel 3) bei Temperaturen unterhalb 0°C

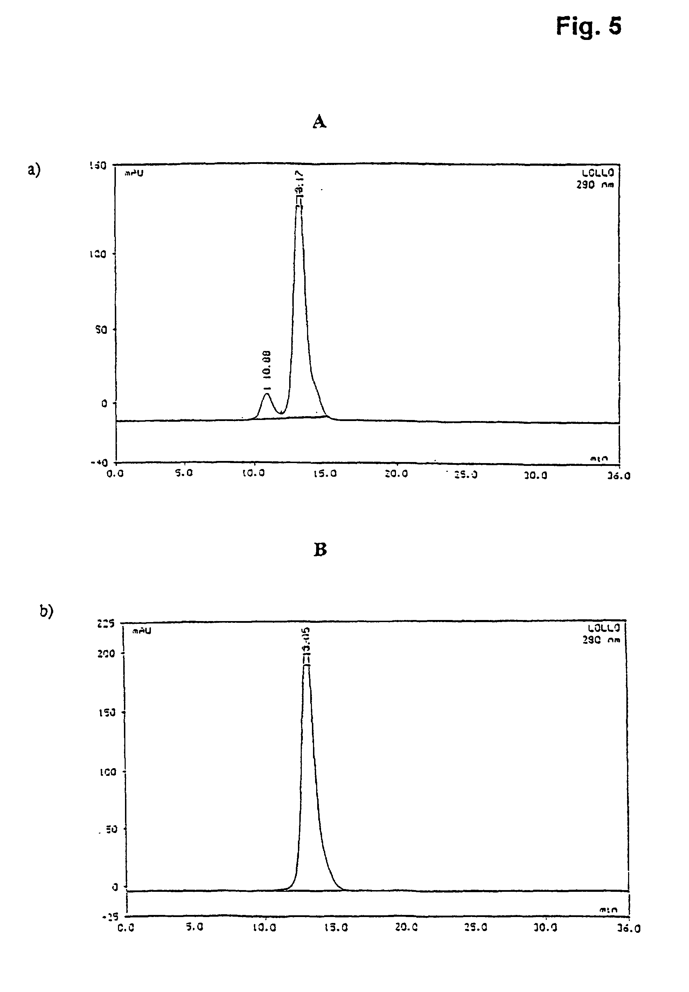

Proteinlösungen wurden in A) 10 mM KP, 150 mM NaCl, pH 7,0 und B) in 100 mM KP, pH 7,2 für mehrere Wochen bei -20°C gelagert. Die Analyse löslicher Aggregate und nativen Proteins erfolgte durch Size Exclusion HPLC (Fig. 5). Erfindungsgemäß treten im NaCl-freien Puffer deutlich weniger Proteinaggregate auf als im NaCl-haltigen Puffer. Dies wird vor allem dadurch bedingt, daß beim NaCl-freien Puffer eine Verschiebung des pH-Wertes weitgehend verhindert wird und die Lagertemperatur deutlich unterhalb der eutektischen Temperatur liegt (siehe auch Beispiel 1 und 2).

### Beispiel 6:

### Partikelbildung in Proteinlösungen nach Einfrier/Auftaustreß

Die Antikörper MAK L-Selektin, MAK HBV, MAK PDGF-R und MAK LNGF-R in verschiedenen Puffern wurden auf Partikelgehalt vor bzw. nach Einfrier/Auftaustreß (6 x Einfrieren/Auftauen) analysiert (Accu Sizer, Particle Sizing Systems) (Ergebnisse vgl. Tabelle 1, Cₚᵣₒₜ : Proteinkonzentration). Angegeben sind die Partikel der Größe 2-400 µm pro ml.
Es wird deutlich, daß die Partikelbildung durch Verwendung von natriumfreien Kaliumphosphatpuffern (KP) erfindungsgemäß unterdrückt wird. Dieser Effekt kann durch die Zugabe eines nichtionischen Detergens gesteigert werden.

**Tabelle 1**

| **MAK L- Selektin in Puffer** | **C**_{**Prot**} **[mg/ml]** | **Partikel/ml ohne Belastung 2-400 µm** | **Partikel/ml 6 x Einfrieren/Auftauen 2- 400 µm** |
|---|---|---|---|
| 10 mM KP, 150 mM NaCl, pH 7.2 | 21.40 | 875 | 6245 |
| 100 mM KP, 0.01% Tween 80, pH 7.2 | 18.50 | 276 | 332 |

| **MAK HBV in Puffer** | **C**_{**Prot**} **[mg/ml]** | **Partikel/ml ohne Belastung 2- 400 µm** | **Partikel/ml 6 x Einfrieren/Auftauen 2- 400 µm** |
|---|---|---|---|
| 10 mM KP, 30 mM NaCl, pH 6.6 | 17.85 | 544 | 19085 |
| 100 mM KP, 0.01% Tween 80, pH 7.2 | 18.30 | 740 | 695 |

| **MAK PDGF- R in Puffer** | **C**_{**Prot**} **[mg/ml]** | **Partikel/ml ohne Belastung 2-400 µm** | **Partikel/ml 6 x Einfrieren/Auftauen 2- 400 µm** |
|---|---|---|---|
| 10 mM KP, 150 mM, NaCl, pH 7.2 | 1.70 | 130 | 33795 |
| 50 mM KP, 0.01 % Tween 80, pH 7.2 | 1.70 | 691 | 677 |

| **MAK LNGF- R in Puffer** | **C**_{**Prot**} **[mg/ml]** | **Partikel/ml ohne Belastung 2- 400 µm** | **Partikel/ml 6 x Einfrieren/Auftauen 2- 400 µm** |
|---|---|---|---|
| 10 mM KP, 150 mM NaCl, pH 7.2 | 1.70 | 690 | 28915 |
| 50 mM KP, 0.01 % Tween 80, pH 7.2 | 1.70 | 1164 | 1257 |

### Referenzliste

DE 26 52 636
EP-A 0 018 609
EP-A 0 025 275
EP-A 0 314 095
EP-A 0 315 968
EP-A 0 318 081
EP-A 0 599 344
GB 8514349
Mendoza, J.A. Biotechnol. Tech. 10 (1991) 535 - 540
US-Patent 4,808,705
WO 91/15509
WO 93/22335

## Patentansprüche

1. Verbessertes Verfahren zur Verhinderung der Bildung von Proteinaggregaten in einem rekonstituierten Lyophilisat einer pharmazeutischen Zusammensetzung bestehend aus einer wäßrigen gepufferten Lösung eines Proteins, wobei die genannte Lösung aufgetaut, in Kompartimente von injizierbaren Mengen zerteilt und diese Kompartimente lyophilisiert werden, **dadurch gekennzeichnet, daß** die genannte Lösung des Proteins Kaliumphosphatpuffer als Puffersubstanz enthält und das Verhältnis Kalium- zu Natriumionen in der Lösung 10 : 1 oder größer ist.

2. Verfahren nach Anspruch 1, **dadurch gekennzeichnet, daß** die Pufferkonzentration zwischen 10 mmol/l und 300 mmol/l im Kompartiment beträgt.

3. Verfahren nach den Ansprüchen 1 - 2, **dadurch gekennzeichnet, daß** die Osmolarität der rekonstituierten Lösung des Kompartiments zwischen 100 und 500 mOsm, bevorzugt 300 ± 50 mOsm beträgt.

4. Verfahren nach den Ansprüchen 1 - 3 **dadurch gekennzeichnet, daß** die wäßrige gepufferte Lösung im pH-Bereich zwischen 6 - 8 gepuffert ist.

5. Verfahren nach den Ansprüchen 1 - 4, **dadurch gekennzeichnet, daß** die wäßrige gepufferte Lösung ein nichtionisches Detergens enthält.

6. Verfahren nach Anspruch 1 - 5, **dadurch gekennzeichnet, daß** die wäßrige gepufferte Lösung einen Zucker in einer Konzentration von 10 - 100 mg/ml enthält.

7. Verfahren nach den Ansprüchen 1 - 6, **dadurch gekennzeichnet, daß** das Protein ein Antikörper ist.

8. Aggregatarme, schmelzbare feste Lagerform eines Proteins, **dadurch gekennzeichnet, daß** sie amorph ist, eine eingefrorene Lösung des Proteins mit Kaliumphosphatpuffer als Puffersubstanz enthält und das Verhältnis Kaliumionen zu Natriumionen mindestens 10 : 1 ist, mit der Maßgabe, daß die genannte eingefrorene Lösung kein Polyethylenglycol enthält.

9. Aggregatarme, schmelzbare feste Lagerform eines Antikörpers, **dadurch gekennzeichnet, daß** sie amorph ist, eine eingefrorene Lösung des Proteins mit Kaliumphosphatpuffer als Puffersubstanz enthält und das Verhältnis Kaliumionen zu Natriumionen mindestens 10 : 1 ist.

10. Feste Lagerform eines Proteins nach Anspruch 8 oder eines Antikörpers nach Anspruch 9, **dadurch gekennzeichnet, daß** die Lagerform durch Lyophilisation hergestellt wird.

11. Pharmazeutische Zusammensetzung bestehend aus einer aggregatarmen wäßrigen gepufferten Lösung eines Proteins im pH-Bereich zwischen 6 und 8, **dadurch gekennzeichnet, daß** die Lösung Kaliumphosphatpuffer als Puffersubstanz enthält und
a) das Verhältnis Kalium zu Natriumionen in der Lösung 10 : 1 oder größer ist,
b) die Pufferkonzentration zwischen 10 und 300 mmol/l beträgt
mit der Maßgabe, daß die genannte Zusammensetzung kein Polyethylenglycol enthält.

12. Pharmazeutische Zusammensetzung bestehend aus einer aggregatarmen wäßrigen gepufferten Lösung eines Antikörpers im pH-Bereich zwischen 6 und 8, **dadurch gekennzeichnet, daß** die Lösung Kaliumphosphatpuffer als Puffersubstanz enthält und
a) das Verhältnis Kalium zu Natriumionen in der Lösung 10 : 1 oder größer ist,
b) die Pufferkonzentration zwischen 10 und 300 mmol/l beträgt.

## Claims

1. Improved process for preventing the formation of protein aggregates in a reconstituted lyophilisate of a pharmaceutical composition comprising an aqueous buffered solution of a protein, in which the said solution is thawed, divided up into compartments of injectable amounts and these compartments are lyophilized, **characterized in that** the said solution of the protein contains potassium phosphate buffer as the buffer substance and the ratio of potassium to sodium ions in the solution is 10:1 or higher.

2. Process as claimed in claim 1, **characterized in that** the buffer concentration in the compartment is between 10 mmol/l and 300 mmol/l.

3. Process as claimed in claims 1 - 2, **characterized in that** the osmolarity of the reconstituted solution of the compartment is between 100 and 500 mOsm, preferably 300 ± 50 mOsm.

4. Process as claimed in claims 1 - 3, **characterized in that** the aqueous buffered solution is buffered in the pH range between 6 - 8.

5. Process as claimed in claims 1 - 4, **characterized in that** the aqueous buffered solution contains a non-ionic detergent.

6. Process as claimed in claims 1 - 5, **characterized in that** the aqueous buffered solution contains a sugar at a concentration of 10- 100 mg/ml.

7. Process as claimed in claims 1 - 6, **characterized in that** the protein is an antibody.

8. Meltable solid storage form of a protein with a low aggregate content, **characterized in that** it is amorphous, contains a frozen solution of the protein in potassium phosphate buffer as the buffer substance and the ratio of potassium ions to sodium ions is at least 10:1, provided that the said frozen solution does not contain polyethylene glycol.

9. Meltable solid storage form of an antibody with a low aggregate content, **characterized in that** it is amorphous, contains a frozen solution of the protein in potassium phosphate buffer as the buffer substance and the ratio of potassium ions to sodium ions is at least 10:1.

10. Solid storage form of a protein as claimed in claim 8 or of an antibody as claimed in claim 9, **characterized in that** the storage form is produced by lyophilization.

11. Pharmaceutical composition comprising an aqueous buffered solution of a protein with a low aggregate content in the pH range between 6 and 8, **characterized in that** the solution contains potassium phosphate buffer as the buffer substance and
a) the ratio of potassium to sodium ions in the solution is 10:1 or higher,
b) the buffer concentration is between 10 and 300 mmol/l
provided that the said composition does not contain polyethylene glycol.

12. Pharmaceutical composition comprising an aqueous buffered solution of an antibody with a low aggregate content in the pH range between 6 and 8, **characterized in that** the solution contains potassium phosphate buffer as the buffer substance and
a) the ratio of potassium to sodium ions in the solution is 10:1 or higher,
b) the buffer concentration is between 10 and 300 mmol/l.

## Revendications

1. Procédé amélioré pour empêcher la formation d'agrégats protéiques dans un lyophilisat reconstitué d'une préparation pharmaceutique constituée d'une solution tamponnée aqueuse d'une protéine, procédé dans lequel on décongèle la solution mentionnée, on la subdivise en compartiments de quantités injectables et on lyophilise ces compartiments, **caractérisé en ce que** la solution mentionnée de la protéine contient en tant que substance tampon un tampon phosphate de potassium, et le rapport des ions potassium aux ions sodium dans la solution est de 10:1 ou plus.

2. Procédé selon la revendication 1, **caractérisé en ce que** la concentration du tampon dans le compartiment est comprise entre 10 et 300 mmol/l.

3. Procédé selon les revendications 1-2, **caractérisé en ce que** l'osmolarité de la solution reconstituée du compartiment est comprise entre 100 et 500 mOsm, et est de préférence de 300 ± 50 mOsm.

4. Procédé selon les revendications 1-3, **caractérisé en ce que** la solution tamponnée aqueuse est tamponnée sur la plage de pH de 6-8.

5. Procédé selon les revendications 1-4, **caractérisé en ce que** la solution tamponnée aqueuse contient un détergent non ionique.

6. Procédé selon les revendications 1-5, **caractérisé en ce que** la solution tamponnée aqueuse contient un sucre à une concentration de 10-100 mg/ml.

7. Procédé selon les revendications 1-6, **caractérisé en ce que** la protéine est un anticorps.

8. Forme stockable d'une protéine, solide, fusible et pauvre en agrégats, **caractérisée en ce qu'**elle est amorphe, qu'elle contient une solution congelée de la protéine avec un tampon phosphate de potassium servant de substance tampon, le rapport des ions potassium aux ions sodium étant d'au moins 10:1, du moment que la solution congelée mentionnée ne contient pas de polyéthylèneglycol.

9. Forme stockable d'un anticorps, solide, fusible et pauvre en agrégats, **caractérisée en ce qu'**elle est amorphe, elle contient une solution congelée de la protéine avec un tampon phosphate de potassium servant de substance tampon, et le rapport des ions potassium aux ions sodium est d'au moins 10:1.

10. Forme stockable solide d'une protéine selon la revendication 8 ou d'un anticorps selon la revendication 9, **caractérisée en ce que** la forme stockable est préparée par lyophilisation.

11. Composition pharmaceutique constituée d'une solution tamponnée aqueuse d'une protéine, pauvre en agrégats, sur la plage de pH de 6 à 8, **caractérisée en ce que** la solution contient en tant que substance tampon un tampon phosphate de potassium, et
a) le rapport des ions potassium aux ions sodium dans la solution est de 10:1 ou plus,
b) la concentration du tampon est de 10 à 300 mmol/l,
du moment que la composition mentionnée ne contient pas de polyéthylèneglycol.

12. Composition pharmaceutique constituée d'une solution tamponnée aqueuse d'un anticorps pauvre en agrégats, sur la plage de pH de 6 à 8, **caractérisée en ce que** la solution contient en tant que substance tampon un tampon phosphate de potassium, et
a) le rapport des ions potassium aux ions sodium dans la solution est de 10:1 ou plus,
b) la concentration du tampon est comprise entre 10 et 300 mmol/l.
